# EUROPÄISCHE PATENTSCHRIFT

(11) **EP 0 820 471 B1**
(45) Veröffentlichungstag und Bekanntmachung des Hinweises auf die Patenterteilung: **24.01.2001**
(21) Anmeldenummer: 96909024.0
(22) Anmeldetag: 09.04.1996
(51) Int. Cl.: C07K 16/18, C12P 21/08, G01N 33/577, G01N 33/68, C12N 5/20

(54) **ANTIKÖRPER GEGEN HERZMUSKEL-ACTIN**
ANTIBODIES AGAINST HEART MUSCLE ACTIN
ANTICORPS ANTI-ACTINE DU MYOCARDE

(30) Priorität: 10.04.1995 DE 19513595
(43) Veröffentlichungstag der Anmeldung: 28.01.1998
(73) Patentinhaber: PROGEN Biotechnik GmbH, 69123 Heidelberg (DE)
(72) Erfinder: FRANKE, Werner, Wilhelm, D-69117 Heidelberg (DE); STUMPP, Sabine, D-69120 Heidelberg (DE); STEHR, Sabine, D-69254 Malsch (DE)
(74) Vertreter: Schüssler, Andrea, Dr.
(86) Internationale Anmeldenummer: DE9600620
(87) Internationale Veröffentlichungsnummer: WO9632417

(56) Entgegenhaltungen:
- INTERNATIONAL JOURNAL OF CARDIOLOGY, Bd. 38, Nr. 1, Januar 1993, AMSTERDAM, NL, Seiten 49-55, XP000578319 A.E. AR NEGA ET AL.: "CIRCULATING ALPHA-ACTIN PROTEIN IN ACUTE MYOCARDIAL INFARCTION"
- JOURNAL OF MOLECULAR AND CELLULAR CARDIOLOGY, Bd. 25, Nr. 1, Januar 1993, LONDON, GB, Seiten 15-22, XP000578297 A.E. AR NEGA ET AL.: "CIRCULATING ALPHA-ACTIN IN ANGINA PECTORIS."
- PEPTIDE RESEARCH, Bd. 2, Nr. 6, November 1989, NATRICK, GB, Seiten 367-372, XP002010880 V. HANIN ET AL.: "PRODUCTION OF OLIGOCLONAL ANTIBODIES DIRECTED TO THE N-TERMINAL OF SMOOTH MUSCLE ALPHA ACTIN USING PEPTIDYL-POLYACRYLIC RESINS AS DIRECT IMMUNOGENS."
- "SIGMA IMMUNO CHEMICALS, 1992 KATALOG" 1992 , SIGMA CHEMIE GMBH , DEISENHOFEN, DE XP002010881 siehe A 2172 siehe Seite 48, mittlere Spalte

## Beschreibung

Die vorliegende Erfindung betrifft Antikörper gegen Herzmuskel-Actin, Verfahren zur Herstellung solcher Antikörper und ihre Verwendung.

Es sind Verfahren bekannt, einen Herzinfarkt durch Bestimmung der Herzmuskelspezifischen Kreatin-Phosphokinase bzw. durch Bestimmung der Serum-Lactatdehydrogenase nachzuweisen. Erstere Bestimmung kann allerdings erst drei Stunden nach einem Herzinfarkt erfolgen. Auch liegt die Kreatin-Phosphokinase nach einem Tag nur noch zu max. 50 % vor. Desweiteren ist die Serum-Lactatdehydrogenase kein Herz-spezifisches Enzym. Vorstehende Bestimmungen eignen sich also nur bedingt zum Nachweis eines Herzinfarkts oder einer anderen Läsion des Herzmuskels.

Der vorliegenden Erfindung liegt somit die Aufgabe zugrunde, ein Mittel bereitzustellen, mit dem ein Herzinfarkt oder eine andere Läsion des Herzmuskels spezifisch und zuverlässig nachgewiesen werden kann.

Erfindungsgemäß wird dies durch Antikörper erreicht, die gegen Herzmuskel-Actin gerichtet sind.

Aus Versuchen der Anmelderin geht hervor, daß bei einem Herzinfarkt Herzmuskel-Actin freigesetzt wird. Dieses Actin unterscheidet sich in wenigen Aminosäuren vom Actin des Skelettmuskels bzw. jenem der glatten Muskulatur (vgl. Vandekerckhove, J. und Weber, K., Differentiation 14, (1979), Seiten 123-133). Erfindungsgemäß werden diese Unterschiede genutzt, Antikörper gegen Herzmuskel-Actin zu richten.

Solche Antikörper können polyklonal oder monoklonal sein, wobei monoklonale Antikörper bevorzugt sind. Die Antikörper können aus jeglichem Tier oder dem Menschen erhalten sein, wobei für polyklonale Antikörper Kaninchen und für monoklonale Mäuse bevorzugt sind.

Ferner können die Antikörper synthetisch sein, wobei ihnen ggfs. Teile, die für die Erkennung des Herzmuskel-Actins nicht notwendig sind, ganz oder teilweise fehlen bzw. diese Teile durch andere ersetzt sind, die den Antikörpern weitere günstige Eigenschaften verleihen.

Der Ausdruck "Herzmuskel-Actin" umfaßt Herzmuskel-Actin jeglicher Art und Abstammung sowie Fragmente davon. Es kann von Mensch oder Tier stammen. Ferner kann das Herzmuskel-Actin in freier oder komplexierter Form vorliegen.

Bevorzugte Antikörper der vorliegenden Erfindung, nämlich die monoklonalen Maus-Antikörper Ac1-12.3.1 und Ac1-20.4.2 wurden bei der DSM unter den Nummern DSM ACC 2208 bzw. DSM ACC 2209 am 22. März 1995 hinterlegt.

Erfindungsgemäße Antikörper können nach üblichen Verfahren hergestellt werden. Sollen polyklonale bzw. monoklonale Antikörper hergestellt werden, ist es günstig, Tiere, insbesondere Kaninchen für erstere und Mäuse für letztere Antikörper, mit einem vorstehenden Herzmuskel-Actin und/oder Fragmenten davon, insbesondere Fragmenten des N- oder C-Terminus von Herzmuskel-Actin, zu immunisieren. Der Ausdruck "Fragmenten davon" umfaßt auch synthetische Peptide, die Teilsequenzen, insbesondere des N- oder C-Terminus, von Herzmuskel-Actin aufweisen. Vorteilhaft kann es auch sein, die Tiere mit einem Gemisch aus Herzmuskel-Actinen und/oder Fragmenten davon zu immunisieren. Weitere "Booster" der Tiere können mit dem oder den gleichen Herzmuskel-Actinen und/oder Fragmenten davon erfolgen. Auch können andere Herzmuskel-Actine und/oder Fragmente davon oder eine Kombination aus diesen und dem oder den vorhergehenden Herzmuskel-Actinen und/oder Fragmenten davon für "Booster" verwendet werden.

Die polyklonalen Antikörper können dann aus dem Serum der Tiere erhalten werden. Für die monoklonalen Antikörper werden Milzzellen der Tiere mit Myelomzellen fusioniert.

Zur Herstellung von synthetischen Antikörpern kann z.B. von vorstehend erhaltenen, monoklonalen Antikörpern ausgegangen werden. Hierzu bietet sich an, die Antigen-Bindungsregionen der monoklonalen Antikörper zu analysieren und die für die spezifische Herzmuskel-Actin-Erkennung notwendigen und nicht notwendigen Teile zu identifzieren. Die notwendigen Teile können dann modifiziert und die nicht notwendigen ganz oder teilweise eliminiert bzw. durch Teile ersetzt werden, die den Antikörpern weitere günstige Eigenschaften verleihen. Auch können Teile außerhalb der Bindungsregionen der Antikörper modifiziert, eliminiert oder ersetzt werden. Der Fachmann weiß, daß sich für vorstehende Maßnahmen insbesondere die DNA-Rekombinationstechnologie eignet. Diese ist ihm bestens vertraut.

Erfindungsgemäße Antikörper zeichnen sich dadurch aus, daß sie Herzmuskel-Actin bzw. Fragmente davon spezifisch erkennen. Die Antikörper eignen sich daher zum schnellen und zuverlässigen Nachweis von Herzerkrankungen, bei denen Herzmuskel-Actin freigesetzt wird. Diese Erkrankungen umfassen insbesondere den Herzinfarkt.

Der Nachweis vorstehender Erkrankungen kann durch beliebige Nachweisverfahren, insbesondere einen Western Blot, einen ELISA, eine Immunpräzipitation oder durch Immunfluoreszenz, erfolgen. Hierzu können die erfindungsgemäßen Antikörper, wenn es angebracht ist, markiert sein oder in Kombination mit markierten gegen sie gerichteten Antikörpern eingesetzt werden. Desweiteren können vorstehende Antikörper in ein Biosensor-Verfahren eingesetzt werden.

Erfindungsgemäß werden auch Kits bereitgestellt, die vorstehende Antikörper zusammen mit Trägermaterialien und üblichen Hilfsstoffen, wie Puffer, enthalten.

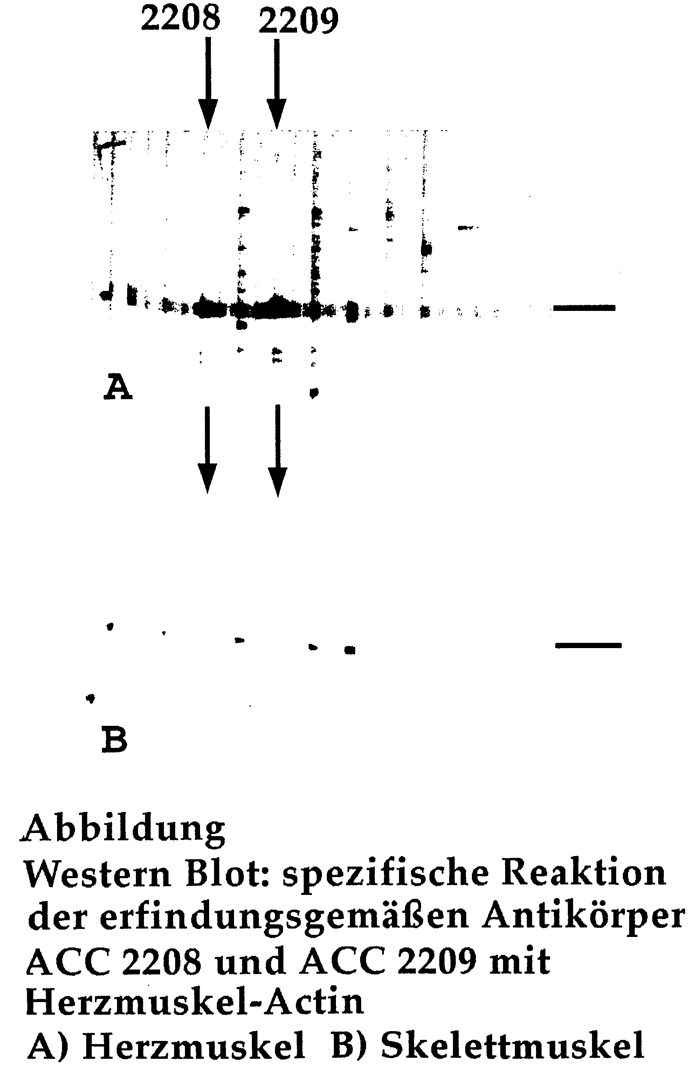

### Kurze Beschreibung der Zeichnung:

Die Figur zeigt einen Western Blot, in dem erfindungsgemäße Antikörper spezifisch Herzmuskel-Actin gegenüber Skelettmuskel-Actin erkennen. Die jeweilige Position des Actins ist am rechten Rand der Figur durch je einen großen Pfeil angezeigt. In den Spuren 7 bzw. 11 von (a) ist die Reaktion mit Herzmuskel-Actin und in den Spuren 7 bzw. 11 von (b) die Nicht-Reaktion mit Skelettmuskel-Actin gezeigt.

Die vorliegende Erfindung wird durch die nachstehenden Beispiele erläutert.

### Beispiel 1: Herstellung von monoklonalen Antikörpern gegen Herzmuskel-Actin

Zur Immunisierung wurden Mäuse des Stammes Balb/c verwendet. Als Antigen wurde ein N-terminales Dekapeptid von Herzmuskel-Actin verwendet. Dieses Dekapeptid enthält die folgende Aminosäuresequenz: DDEETTALVC. Sein N-Terminus weist eine Acetyl-Gruppe und sein C-Terminus eine Amid-Gruppe auf. Das Dekapeptid wurde mit Rinderserumalbumin (BSA) bzw. Keyhole Limpet Hemocyanin (KLH) in üblicher Weise gekoppelt.

### Immunisierung- und "Booster"-Schema:

- Einer Maus wurden 100µg Dekapeptid, konjugiert mit BSA, emulgiert in vollständigem Freund's Adjuvans, verabreicht.
- Es folgten drei Booster-Injektionen mit jeweils 100µg Dekapeptid, konjugiert mit KLH (zweimal) bzw. BSA (einmal) im Wechsel, wobei das Dekapeptid in nicht-vollständigem Freund's Adjuvans emulgiert war.
- Vier Tage vor Entnahme der Milzzellen wurden der Maus 100µg Dekapeptid, konjugiert mit BSA, intraperitoneal verabreicht.
- Die entnommenen Milzzellen wurden mit Maus-Myelomzellen des bekannten Stammes X63-Ag8, 653 fusioniert.

Es wurden monoklonale Antikörper erhalten. Hiervon wurden die mit Ac1-12.3.1 bzw. Ac1-20.4.2 bezeichneten Antikörper bei der DSM unter den Nummern DSM ACC 2208 bzw. DSM ACC 2209 am 22. März 1995 hinterlegt.

### Beispiel 2: Nachweis von Herzmuskel-Actin durch erfindungsgemäße Antikörper

Rinderherz und Kaninchenmuskel wurden jeweils zur Herstellung von Proteinpräparationen verwendet. Dies erfolgte nach einem üblichen Verfahren. Die Proteinpräparationen wurden einer Polyacrylamid-Gelelektrophorese unterzogen und die so aufgetrennten Polypeptide wurden auf eine Nitrocellulosemembran übertragen. Diese wurde dann mit den vorstehenden, 1:10 bzw. 1:50 verdünnten Antikörpern ACC 2208 und ACC 2209 1 Std. bei 37°C inkubiert. Nach mehreren Waschschritten mit PBS (0,05 % Tween 20) und ggfs. einem Waschschritt mit 0,5 M NaCI in PBS wurde ein käuflicher, an alkalische Phosphatase-gekoppelter Anti-Maus-Antikörper (Verdünnung nach Angabe der Hersteller) zugegeben. Nach 30-minütiger Inkubation bei 37°C folgten mehrere Waschschritte mit PBS und anschließend die alkalische Phosphatase-Nachweisreaktion mit Entwicklerlösung (36 µM 5'Bromo-4-chloro-3-indolylphosphat, 400 µM Nitroblau-tetrazolium, 100 mM Tris-HCI, pH 9.5, 100 mM NaCI, 5 mM MgCl₂) bei Raumtemperatur, bis Banden sichtbar waren.

Es zeigte sich, daß die erfindungsgemäßen Antikörper ACC 2208 und ACC 2209 spezifisch Herzmuskel-Actin gegenüber Skelettmuskel-Actin erkennen. Die Spezifität der Antikörper ist dabei besonders hoch, wenn der vorstehende Waschschritt mit 0,5 M NaCI in PBS durchgeführt wird.

## Patentansprüche

1. Antikörper gegen Herzmuskel-Actin, dadurch gekennzeichnet, daß der Antikörper nicht mit Skelettmuskel-Actin reagiert.

2. Antikörper nach Anspruch 1, dadurch gekennzeichnet, daß sie polyklonal sind.

3. Antikörper nach Anspruch 1, dadurch gekennzeichnet, daß sie monoklonal sind.

4. Antikörper nach Anspruch 3, dadurch gekennzeichnet, daß er bei der DSM unter ACC 2208 hinterlegt ist.

5. Antikörper nach Anspruch 3, dadurch gekennzeichnet, daß er bei der DSM unter ACC 2209 hinterlegt ist.

6. Verfahren zur Herstellung eines Antikörpers nach einem der Ansprüche 1-5, dadurch gekennzeichnet, daß ein Tier mit einem Herzmuskel-Actin, einem Fragment davon und/oder einem synthetischen, eine Teilsequenz von Herzmuskel-Actin aufweisenden Peptid immunisiert wird, und
(a) polyklonale Antikörper aus dem Serum des Tieres erhalten werden, oder
(b) monoklonale Antikörper nach Fusion von Milzzellen des Tieres mit Myelomzellen erhalten werden.

7. Verfahren nach Anspruch 6, dadurch gekennzeichnet, daß ein Fragment des N- oder C-Terminus von Herzmuskel-Actin oder ein synthetisches, eine entsprechende Aminosäuresequenz aufweisendes Peptid zur Immunisierung eingesetzt wird.

8. Verfahren nach Anspruch 7, dadurch gekennzeichnet, daß das Fragment die Aminosäuresequenz DDEETTALVC enthält.

9. Verwendung eines Antikörpers nach einem der Ansprüche 1-5 in einem in vitro Verfahren zum Nachweis von Herzerkrankungen, insbesondere Herzinfarkt.

10. Verwendung nach Anspruch 9, wobei das Nachweisverfahren ein Western-Blot, ein ELISA, ein lmmunfluoreszenz-Verfahren, eine Immunpräzipitation oder ein Biosensor-Verfahren ist.

## Claims

1. Antibody against heart muscle actin, characterized in that the antibody does not react with skeletal muscle actin.

2. Antibodies according to claim 1, characterized in that they are polyclonal.

3. Antibodies according to claim 1, characterized in that they are monoclonal.

4. Antibody according to claim 3, characterized in that it has been deposited under ACC 2208 with the DSM.

5. Antibody according to claim 3, characterized in that it has been deposited under ACC 2209 with the DSM.

6. Method for preparing an antibody according to any of claims 1-5, characterized in that an animal is immunized with an heart muscle actin, a fragment thereof and/or a synthetic peptide having a partial sequence of heart muscle actin, and
(a) polyclonal antibodies are obtained from the animal, or
(b) monoclonal antibodies are obtained after fusion of spleen cells of the animal with myeloma cells.

7. Method according to claim 6, characterized in that a fragment of the N- or C-terminus of heart muscle actin or a synthetic peptide having a corresponding amino acid sequence is used for immunisation.

8. Method according to claim 7, characterized in that the fragment contains the amino acid sequence DDEETTALVC.

9. Use of an antibody according to any of claims 1-5 in an in-vitro method for detecting heart diseases, in particular infarction.

10. Use according to claim 9, wherein the detection method is a western blot, an ELISA, an immunofluorescence method, an immunoprecipitation or a biosensor method.

## Revendications

1. Anticorps dirigé contre l'actine du myocarde, caractérisé en ce que l'anticorps ne réagit pas avec l'actine du muscle squelettique.

2. Anticorps selon la revendication 1, caractérisés en ce qu'ils sont polyclonaux.

3. Anticorps selon la revendication 1, caractérisés en ce qu'ils sont monoclonaux.

4. Anticorps selon la revendication 3, caractérisé en ce qu'il a été déposé auprès de la DSM sous le numéro ACC 2208.

5. Anticorps selon la revendication 3, caractérisé en ce qu'il a été déposé auprès de la DSM sous le numéro ACC 2209.

6. Procédé de préparation d'un anticorps selon l'une des revendications 1 à 5, caractérisé en ce qu'on immunise un animal avec une actine du myocarde, un fragment de celle-ci et/ou un peptide synthétique présentant une séquence partielle d'actine du myocarde et
(a) on obtient des anticorps polyclonaux à partir du sérum de l'animal ou
(b) on obtient des anticorps monoclonaux après fusion de cellules de la rate de l'animal avec des cellules de myélome.

7. Procédé selon la revendication 6, caractérisé en ce qu'on utilise un fragment de l'extrémité N- ou C-terminale d'actine du myocarde ou un peptide synthétique présentant une séquence correspondante d'amino-acides pour l'immunisation.

8. Procédé selon la revendication 7, caractérisé en ce que le fragment contient la séquence d'amino-acides DDEETTALVC.

9. Utilisation d'un anticorps selon l'une des revendications 1 à 5 dans un procédé *in vitro* pour détecter des maladies cardiaques, en particulier un infarctus du myocarde.

10. Utilisation selon la revendication 9, le procédé de détection étant un procédé *Western Blot,* un test ELISA, un procédé par immunofluorescence, une immunoprécipitation ou un procédé avec un biocapteur.
